# EUROPEAN PATENT APPLICATION

(11) **EP 1 357 379 A2**
(43) Date of publication of application: **29.10.2003**
(21) Application number: 03251691.6
(22) Date of filing: 19.03.2003
(51) Int. Cl.: G01N 27/49

(54) **Sodium chloride solution humidified sensor**

(30) Priority: 15.04.2002 US 122683
(71) Applicant: Mocon, Inc., Minneapolis, Minnesota 55428 (US)
(72) Inventor: Tuomela, Steve, Ramsey, MN 55303 (US)
(74) Representative: Sturt, Clifford Mark

(57) **Abstract**

A galvanic measuring device for detecting oxygen content in a gas flow, having a sealed gas flow chamber and a layer of sodium chloride (NaCl) saturated sponge (100) material lining the interior of the gas flow chamber, for providing a constant source of humidity to the chamber.

## Description

### Background of the Invention

The present invention relates to oxygen sensors, and more particularly, oxygen sensors having an internal humidification apparatus for extending the useful life of the sensor. Oxygen sensors functioning as coulometric sensors have long been useful in the test and measurement field, as for example, sensors described in U.S. Patent No. 3,223,597 issued Dec. 14, 1965 to Hersch. Such sensors typically use a glass envelope having an inlet and an outlet to permit the flow of a gas therethrough, and an anode and cathode inside the glass envelope, exposed to the flow of the gas. Oxygen molecules in the gas cause a current flow between the cathode and anode, and the current flow is conveyed via wires to an indicator circuit, where the amount of current flow is representative of the amount of oxygen flow through the glass envelope. A solution of potassium hydroxide (KOH) is placed in the envelope, to facilitate the flow of current as described. Eventually, the KOH solution dries out inside the glass envelope, and the device must be recharged with KOH or replaced.

More recently, improvements in sensitivity of instruments of this type have led to the construction of sensors having two such coulometric sensors of the type described above inside a single sealed container, and connected in series gas flow relationship. In order to extend the sensor life, the interior of the single container is at least partially filled with KOH, and the gas flow path includes a section of nafion tubing which is immersed in the KOH solution. Since nafion has the characteristic of being a barrier to oxygen and being permeable to water vapor, the presence of the section of nafion tubing allows water vapor to enter the gas flow path, and to humidify the gas flowing through the flow path. A device of this type is disclosed in U.S. Patent No. 4,973,395, owned by the assignee of the present invention, and incorporated by reference herein.

The downside to at least partially filling the sensor container with KOH, as shown in the '395 patent, is that this material is hazardous and is extremely corrosive to other materials. If the sensor container should develop a leak, the KOH may leak out of the container and corrode any materials or components which might be nearby. This corrosive action could destroy the relatively expensive equipment associated with the sensing and measurement device. Therefore, there is a need to provide the relative humidity needed to conduct many measurement tests without risking the possible leakage of hazardous and corrosive liquids from within the sensor device.

### Summary of the Invention

An oxygen detection device having internal humidification without the need to partially fill the inside of the detector body with hazardous and corrosive material. This is accomplished by lining the interior surface of the device with a material that is saturated with a solution including sodium chloride (NaCl), otherwise known as common table salt. The material is a liquid absorbent material such as a plastic sponge, which retains a saturated solution without creating a dripping of the solution on other interior components.

It is therefore a principal object of the present invention to provide a humidification feature for coulometric detectors which does not involve the use of KOH or other hazardous and corrosive materials.

Other and further objects and advantages of the invention will become apparent from the following specification and claims and with reference to the appended drawings.

### Brief Description of the Drawings

FIG. 1 shows a side cross-sectional view of a detector having the invention installed in the interior;
FIG. 2 shows a top cross-sectional view orthogonal to the view of FIG. 1;
FIG. 3 shows a cross-sectional view taken along the lines 3 - 3 of FIG. 1; and
FIG. 4 shows a wiring diagram of the circuits formed by the invention.

### Description of the Preferred Embodiment

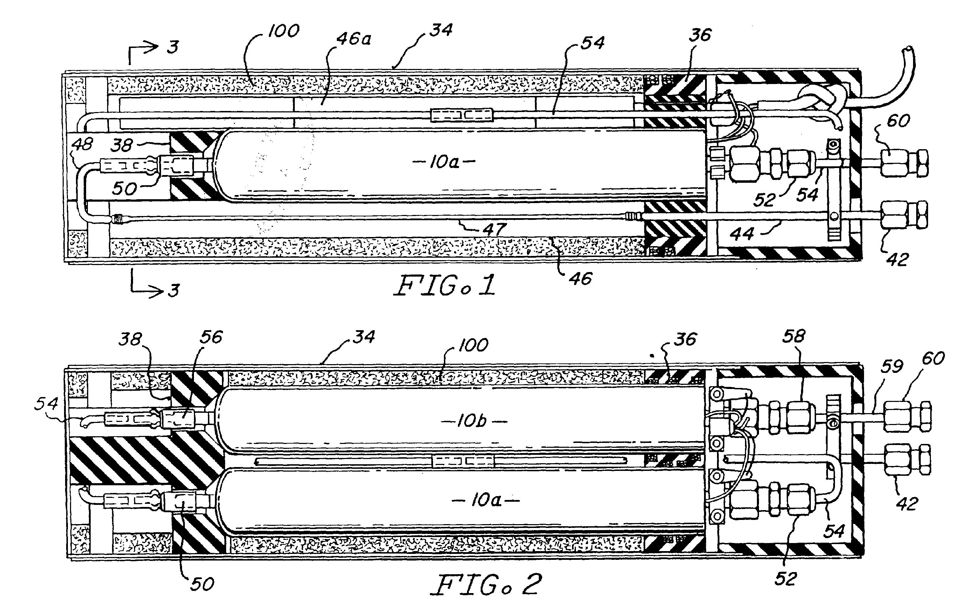

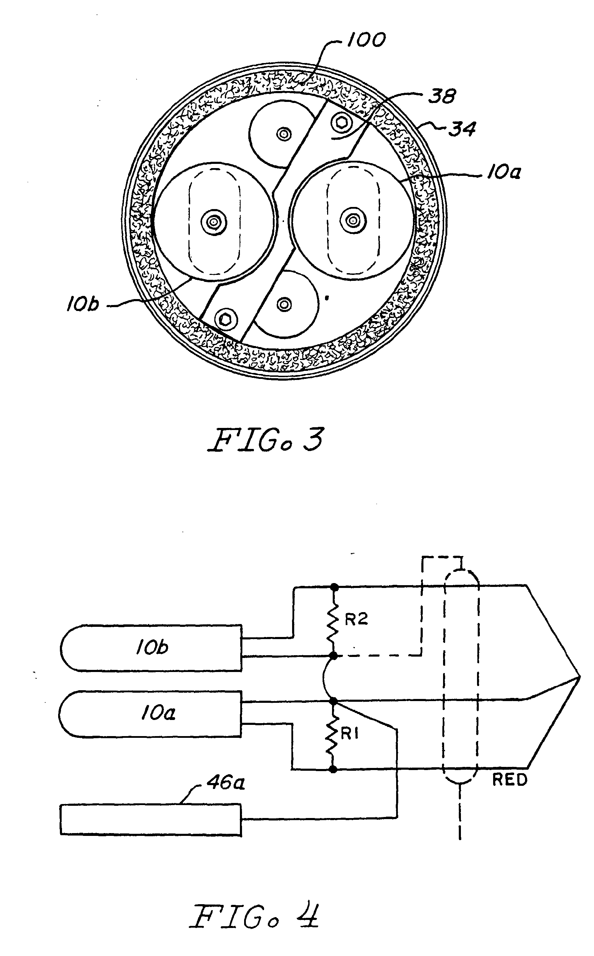

Referring to the drawing figures, like reference characters refer to the same or functionally similar parts of the device illustrated in each of the figures. FIGs. 1 and 2 respectively show a side and top cross-sectional view of an enclosure 34 having a pair of galvanic cells 10a and 10b sealed within the enclosure by a plug 36. They are supported at their inner ends by a spacer 38.

An inlet 42 is adapted for coupling to a source of test gas, and is coupled to a gas conduit 44 which passes through outer wall 43 of enclosure 34. Gas conduit 44 sealably passes through plug 36 and is coupled to a gas humidifier 46, which has an internal nafion tube 47. Nafion has the characteristic that it is permeable to water, and is chemically resistant to and a barrier to KOH. Enclosure 34 also contains an oxygen getter 46a to remove any trace amounts of oxygen which may accumulate inside sealed enclosure 34, particularly in the KOH solution. The name "nafion" is a trademark of the E.I. Dupont de Nemours Company.

The output of nafion tube 47 is coupled to a conduit 48, which is connected to an inlet 50 of galvanic cell 10a. Galvanic cell 10a has an outlet 52 coupled to a further conduit 54 outside of the sealed portion of enclosure 34. Conduit 54 sealably passes through plug 36, and passes through the getter 46a, and is connected to an inlet 56 of galvanic cell 10b. Galvanic cell 10b has an outlet 58 which is coupled to a conduit 59 which passes through the outer wall 43, and is connected to an outlet coupling 60, adapted for connection to an exhaust circuit.

An interior resilient and absorbent pad or sponge 100 is arranged around the inner periphery of enclosure 34, inside the sealed portion of the interior. This sponge is made from a very absorbent material, and is saturated with a sodium chloride (NaCl) solution in water prior to inserting it into the enclosure 34. This provides a continuous source of humidification for so long as any NaCl solution remains in the sponge 100, and maintains a relatively constant relative humidity in the enclosure at about 75%. There is no excessive KOH inside the enclosure 34, beyond the saturated condition that each of the galvanic cells normally require during the manufacturing process; therefore, there is no chance of leakage of KOH from the enclosure, even in the event it becomes cracked or broken during use.

FIG. 3 shows a cross-section view taken along the lines 3 - 3 of FIG. 1. This shows the inner liner sponge 100 wrapped around the entire inside periphery of enclosure 34.

FIG. 4 shows the electrical circuit diagram of the invention, illustrating how the galvanic cells are wired to produce an output electrical signal representative of the oxygen content of the gas passing through the cells. For example, if the value of the resistances R1 and R2 are each selected to be 10,000 ohms (10k) and the test gas is passed through the device at a 10cc per minute flow rate, the useful output signal will be in the microvolt range. A one microvolt change in output voltage is equivalent to an oxygen permeability measurement of 0.0001 cc per square meter per day (cc/m²/day); this is approximately a concentration measurement of 36 parts per trillion. The saturated sponge contained within the sensor enclosure permits a constant control of relative humidity of the test gas, and replenishes water loss which otherwise would occur in the galvanic cells during extended periods of operation.

The present invention may be embodied in other forms without departing from the spirit or essential attributes thereof; and it is, therefore, desired that the present embodiment be considered in all respects as illustrative and not restrictive, reference being made to the appended claims rather than to the foregoing description to indicate the scope of the invention.

## Claims

1. An apparatus for detecting oxygen in a test gas flow through a galvanic cell of the type which develops a current flow between a cell cathode and anode representative of oxygen content in the test gas; comprising:
a. a pair of galvanic cells inside a sealed enclosure, said cells connected in series gas flow arrangement, with an input adapted for connection to a source of test gas and an output adapted for connection to a gas exhaust;
b. means for electrically connecting said galvanic cells to provide an electrical signal representative of the oxygen content of said gas flow; and
c. an absorbent material inside said sealed enclosure, said absorbent material being saturated with a sodium chloride solution and water.

2. The apparatus of claim 1, wherein said absorbent material further comprises a layer applied about the inside surface area of said enclosure.

3. The apparatus of claim 2, further comprising a gas humidifier connected in said series gas flow arrangement, said humidifier having a section of tubing which is permeable to water vapor, and being positioned in said enclosure to receive water vapor from said absorbent material.

4. The apparatus of claim 3, wherein said section of tubing further comprises nafion.

5. The apparatus of claim 4, wherein said absorbent material further comprises a resilient sponge material.

6. An apparatus for detecting oxygen in a test gas flow through a galvanic cell of the type which develops a current flow between a cell cathode and cell anode representative of oxygen content in the test gas, comprising:
a. an enclosure having a sealed interior chamber;
b. at least one galvanic cell in said sealed interior chamber, said at least one galvanic cell having an input outside said chamber for receiving a flow of test gas, and an output for exhausting said flow of test gas;
c. a water permeable section of tubing in said chamber and in series flow arrangement of said test gas;
d. a layer of absorbent sponge material applied against an inside surface of said chamber, said layer having a saturated sodium chloride and water solution; and
e. means for electrically connecting said at least one galvanic cell for providing an electrical signal representative of oxygen content of said test gas.

7. The apparatus of claim 6, wherein said water permeable section of tubing further comprises nafion tubing.
